# EUROPEAN PATENT APPLICATION

(11) **EP 3 432 313 A1**
(43) Date of publication of application: **23.01.2019**
(21) Application number: 17181796.8
(22) Date of filing: 18.07.2017
(51) Int. Cl.: G16H 50/20, G16H 30/40, G16H 50/70, G06N 99/00, G06T 7/00

(54) **TRAINING AN IMAGE ANALYSIS SYSTEM**

(71) Applicant: Koninklijke Philips N.V., 5656 AE Eindhoven (NL)
(72) Inventor: JOCKEL, Sascha Andreas, 5656 AE Eindhoven (NL); BALTRUSCHAT, Ivo, 5656 AE Eindhoven (NL); NICKISCH, Hannes, 5656 AE Eindhoven (NL); SAALBACH, Axel, 5656 AE Eindhoven (NL)
(74) Representative: de Haan, Poul Erik

(57) **Abstract**

There is provided an apparatus (100) and a method of operating the apparatus for training an image analysis system. The apparatus (100) comprises a processor (102) configured to train an image analysis system to analyse medical images using a set of training data stored in a memory, iteratively control a user interface (106) to output a request for user feedback associated with the set of training data stored in the memory (104), and update the set of training data stored in the memory (104) based on user feedback received from the user interface (106) in response to the request. The processor (102) is also configured to retrain the image analysis system based on the updated set of training data stored in the memory (104).

## Description

### Technical Field of the Invention

The invention relates to the field of image analysis and, in particular, to an apparatus for training an image analysis system and a method of operating the apparatus to train the image analysis system.

### Background to the Invention

A core enabling functionality in intelligent medical systems, workstations, and image archives (for example, picture archiving and communication systems PACSs, vendor neutral archives VNAs, etc.) is knowledge about the content of a medical image in terms of anatomical structures, the orientation of anatomical structures, the presence of implants, the presence of diseases, the presence of defects and applied processing parameters. An existing mechanism that is used in acquiring such knowledge is the use of deep neural networks. Image classification and regression methods based on deep neural networks are a popular choice for classifying natural images and natural languages, and can be applied to results over a broad range of medical imaging applications.

An example of a deep learning mechanism for classifying data using machine learning is provided in US 9,324,022, which discloses that a deep learning model can be trained based on a plurality of classifiers and sets of training data and/or testing data. However, although the classifiers may be tuned by a user in the disclosed system, the system still does not achieve a high enough degree of certainty in the classifications that it provides, which means that the classifications provided by the disclosed system can be inaccurate.

There is thus a need for an improved method and apparatus for training an image analysis system.

### Summary of the Invention

As noted above, a limitation with existing approaches is that the existing approaches do not provide an adequate degree of certainty in the results that they can provide, which means that the results can be inaccurate. It would thus be valuable to have a method and apparatus for training an image analysis system, which overcomes the existing problems.

Therefore, according to a first aspect, there is provided an apparatus for training an image analysis system. The apparatus comprises a processor configured to train an image analysis system to analyse medical images using a set of training data stored in a memory, iteratively control a user interface to output a request for user feedback associated with the set of training data stored in the memory and update the set of training data stored in the memory based on user feedback received from the user interface in response to the request. The processor is also configured to retrain the image analysis system based on the updated set of training data stored in the memory.

In some embodiments, the set of training data may comprise at least one medical image and one or more dependent variables associated with at least one medical image. In some embodiments, the one or more dependent variables may be indicative of a property associated with the at least one medical image.

In some embodiments, the initiated request for user feedback may comprise any one or more of a request for one or more dependent variables to associate with at least one medical image of the set of training data stored in the memory, a request to confirm one or more dependent variables associated with at least one medical image of the set of training data stored in the memory, and a request to correct one or more dependent variables associated with at least one medical image of the set of training data stored in the memory. In some embodiments, the one or more dependent variables may be indicative of a property associated with the at least one medical image.

In some embodiments, the processor may be configured to determine a confidence level for the retrained image analysis system. In some embodiments, the processor may be is configured to control the user interface to output a further request for user feedback associated with the set of training data stored in the memory if the determined confidence level for the retrained image analysis system is determined to be below a minimum threshold.

In some embodiments, the processor may be configured to analyse one or more medical images using the retrained image analysis system. In some embodiments, the processor may be configured to select one or more parameters for image processing to apply to at least one medical image based on the analysis of the at least one medical image using the retrained image analysis system. In some embodiments, the processor may be configured to adjust at least one medical image based on the analysis of the at least one medical image using the retrained image analysis system. In some embodiments, the processor may be configured to adjust the at least one of the medical image by rotating the least one medical image to alter an orientation of the at least one medical image based on the analysis of the at least one medical image using the retrained image analysis system.

In some embodiments, the processor may be configured to analyse the one or more medical images by assigning a classification to one or more medical images using the retrained image classification system and/or performing a regression analysis on one or more medical images using the retrained image classification system. In some embodiments, the processor may be configured to analyse the one or more medical images by assigning a classification to one or more medical images using the retrained image classification system, detect a misclassification of at least one medical image classified using the retrained image classification system and control the user interface to output a request for a user input to assign a classification to the at least one misclassified medical image.

In some embodiments, the processor may be further configured to receive at least one medical image and one or more dependent variables associated with the at least one medical image and control the memory to add the at least one received medical image and the one or more received dependent variables associated with the at least one medical image to the set of training data stored in the memory. In some embodiments, the dependent variables may be indicative of a property associated with the at least one medical image.

In some embodiments, the image analysis system may comprise at least one deep neural network.

According to a second aspect, there is provided a method of operating an apparatus comprising a processor to train an image analysis system. The method comprises training an image analysis system to analyse medical images using a set of training data stored in a memory, iteratively controlling a user interface to initiate a request for user feedback on the set of training data stored in the memory, updating the set of training data stored in the memory based on user feedback from the user interface received in response to the request and retraining the image analysis system based on the updated set of training data stored in the memory.

According to a third aspect, there is provided a computer program product comprising a computer readable medium, the computer readable medium having computer readable code embodied therein, the computer readable code being configured such that, on execution by a suitable computer or processor, the computer or processor is caused to perform the method as described above.

According to the aspects and embodiments described above, the limitations of existing techniques are addressed. In particular, according to the above-described aspects and embodiments, a request for user feedback associated with the set of training data is iteratively output such that the set of training data can be continually updated based on user feedback received in response to the requests, thereby enabling the image analysis system to be retrained based on a continually updated set of training data. Thus, in effect, an active learning system is provided that has a constantly improving accuracy. In this way, it is possible to achieve a higher degree of certainty (confidence level) in an image analysis system that is retrained in the manner described above.

There is thus provided an improved method and apparatus for training an image analysis system, which overcomes the existing problems.

### Brief Description of the Drawings

For a better understanding of the invention, and to show more clearly how it may be carried into effect, reference will now be made, by way of example only, to the accompanying drawings, in which:
Figure 1 is a block diagram of an apparatus for training an image analysis system according to an embodiment;
Figure 2 illustrates a method for training an image analysis system according to an embodiment;
Figure 3 illustrates the apparatus for training an image analysis system in use according to an embodiment; and
Figure 4 illustrates a method for training an image analysis system according to an example embodiment.

### Detailed Description of the Embodiments

As noted above, there is provided an improved method and apparatus for training an image analysis system, which overcomes the existing problems.

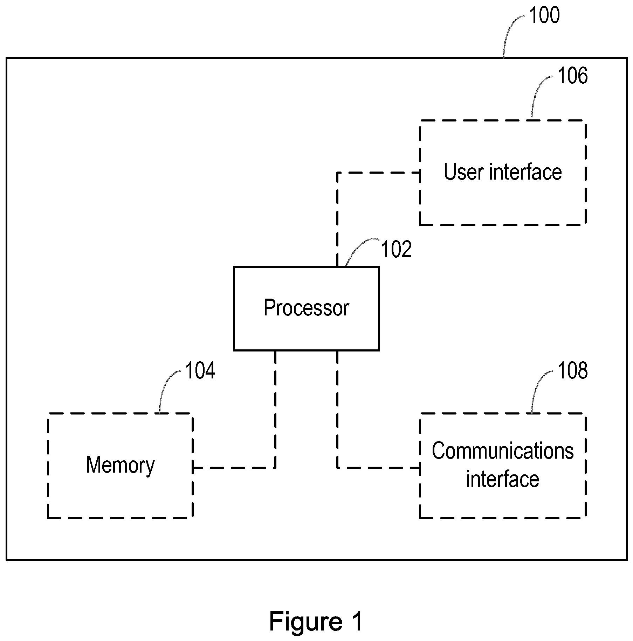

**Figure 1** shows a block diagram of an apparatus 100 according to an embodiment that can be used for training an image analysis system.

With reference to Figure 1, the apparatus 100 comprises a processor 102 that controls the operation of the apparatus 100 and that can implement the method described herein. The processor 102 can comprise one or more processors, processing units, multi-core processors or modules that are configured or programmed to control the apparatus 100 in the manner described herein. In particular implementations, the processor 102 can comprise a plurality of software and/or hardware modules that are each configured to perform, or are for performing, individual or multiple steps of the method described herein.

Briefly, the processor 102 of the apparatus 100 is configured to train an image analysis system to analyse medical images using a set of training data stored in a memory 104. The processor 102 of the apparatus 100 is also configured to iteratively control a user interface 106 to output a request for user feedback associated with the set of training data stored in the memory 104 and update the set of training data stored in the memory 104 based on user feedback received from the user interface 106 in response to the request. The processor 102 is further configured to retrain the image analysis system based on the updated set of training data stored in the memory 104.

The medical images that the image analysis system is trained to analyse can be any type of medical images, or any combination of medical images. Examples of medical images include, but are not limited to, computed tomography (CT) images, magnetic resonance (MR) images, ultrasound (US) images, x-ray images, fluoroscopy images, positron emission tomography (PET) images, single photon emission computed tomography (SPECT) images, nuclear medicine images, or any other modality of medical images, or any combination of modalities of medical images.

The image analysis system referred to herein may also be referred to as an image processing system. The image analysis system can be any generic image analysis system. For example, the image analysis system can comprise at least one image analysis network. In an example embodiment, the image analysis system can comprise at least one deep neural network. Thus, according to some embodiments, the image analysis system can be based on deep learning technology. A person skilled in the art will be aware of deep learning technology and will also be aware of various methods by which to apply deep learning technology. In some embodiments where the image analysis system comprises at least one deep neural network, the image analysis system can comprise a stack (or a set) of deep neural networks. A technical benefit associated with using a deep neural network is that it is not necessary to train the deep neural network from scratch for a particular application and, instead, existing pre-trained deep neural networks can be used and refined (for example, on small training corpora) to transfer the representation to a relevant medical task. In some embodiments, the at least one deep neural network (for example, the stack of deep neural networks) can automatically and constantly monitor the performance of the image analysis system on an existing set of training data and/or a validation (for example, test) set of training data.

The image analysis system referred to herein can be any type of image analysis system. Examples of an image analysis system include, but are not limited to, an image classification system for classifying medical images, an image regression system for performing image regression tasks on the medical images, an image prediction system for providing predictions relating the medical images, a machine learning system for performing machine learning on medical images, or any other type of image analysis system, or any combination of types of image analysis system. For example, in some embodiments, the image analysis system may be a system for classifying images and performing image regression, or a system for performing any other combination of image analysis techniques.

As illustrated in Figure 1, in some embodiments, the apparatus 100 can comprise a memory 104. Thus, according to some embodiments, the set of training data that is used to train the image analysis system may be stored in the memory 104 of the apparatus 100. Alternatively or in addition, in some embodiments, the set of training data may be stored in a memory external to (i.e. separate to or remote from) the apparatus 100, such as a memory of another apparatus or device. The memory 104 of the apparatus 100 can be configured to store program code that can be executed by the processor 102 to perform the method described herein. In some embodiments, alternatively or in addition to the set of training data, the memory 104 of the apparatus 100 can be used to store other data (or information) acquired or made by the processor 102 of the apparatus 100 or acquired from any interfaces, memories or devices that are external to the apparatus 100. The processor 102 of the apparatus 100 may be configured to control the memory 104 to store such data (or information). Similarly, in some embodiments, the processor 102 can be configured to control the memory 104 to store one or more sets of training data for use in the method described herein.

In some embodiments, as illustrated in Figure 1, the apparatus 100 may comprise a user interface 106. Thus, according to some embodiments, the user interface 106 that the processor 102 of the apparatus 100 is configured to iteratively control to output the request for user feedback may be the user interface 106 of the apparatus 100. Alternatively or in addition, in some embodiments, the user interface 106 that the processor 102 of the apparatus 100 is configured to iteratively control to output the request for user feedback may be external to (i.e. separate to or remote from) the apparatus 100, such as a user interface of another apparatus or device. The user interface 106 of the apparatus may be for use in providing a user of the apparatus 100 with information resulting from the method described herein. The processor 102 of the apparatus 100 may be configured to control the user interface 106 to provide the information resulting from the method described herein. Alternatively or in addition, the user interface 106 of the apparatus 100 may be configured to receive a user input. In other words, the user interface 106 of the apparatus 100 may allow a user of the apparatus 100 to manually enter instructions, data, or information. The processor 102 of the apparatus 100 may be configured to acquire the user input from the user interface 106.

A user interface as referred to herein may be any user interface that enables the rendering (or output or display) of data (or information) to a user of the apparatus 100. Alternatively or in addition, a user interface as referred to herein may be any user interface that enables a user of the apparatus 100 to provide a user input, interact with and/or control the apparatus 100. For example, a user interface as referred to herein can comprise one or more switches, one or more buttons, a keypad, a keyboard, a touch screen or an application (for example, on a tablet or smartphone), a display screen, a graphical user interface (GUI), or other visual rendering component, one or more speakers, one or more microphones or any other audio component, one or more lights, a component for providing tactile feedback (e.g. a vibration function), or any other user interface component, or combination of user interface components.

In some embodiments, as illustrated in Figure 1, the apparatus 100 may also comprise a communications interface (or circuitry) 108 for enabling the apparatus 100 to communicate with any interfaces, memories and devices that are internal or external to the apparatus 100. The communications interface 108 may communicate with any interfaces, memories and devices wirelessly or via a wired connection. For example, the communications interface 108 may communicate with the memory 104 of the apparatus 100 or any other memory wirelessly or via a wired connection. Similarly, the communications interface 108 may communicate with the user interface 106 of the apparatus 100 or any other user interface wirelessly or via a wired connection.

It will be appreciated that Figure 1 only shows the components required to illustrate this aspect of the apparatus and, in a practical implementation, the apparatus 100 may comprise additional components to those shown. For example, the apparatus 100 may comprise a battery or other power supply for powering the apparatus 100 or means for connecting the apparatus 100 to a mains power supply.

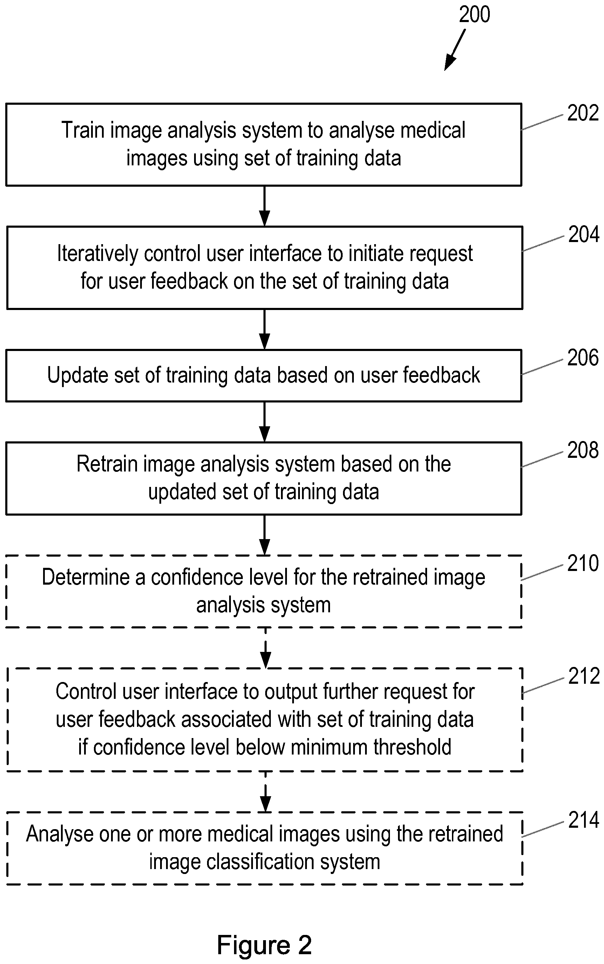

**Figure 2** illustrates a method 200 of operating an apparatus comprising a processor to train an image analysis system according to an embodiment. The illustrated method 200 can generally be performed by or under the control of the processor 102 of the apparatus 100.

Briefly, with reference to Figure 2, the method comprises training an image analysis system to analyse medical images using a set of training data stored in a memory (at block 202 of Figure 2), iteratively controlling a user interface to initiate a request for user feedback on the set of training data stored in the memory (at block 204 of Figure 2) and updating the set of training data stored in the memory based on user feedback received from the user interface in response to the request (at block 206 of Figure 2). The method also comprises retraining the image analysis system based on the updated set of training data stored in the memory (at block 208 of Figure 2). In some embodiments, the method 200 of Figure 2 may be repeated in an iterative manner. In some embodiments, the method 200 of Figure 2 may be an automated method.

In more detail, at block 202 of Figure 2, the image analysis system is trained to analyse medical images using the set of training data stored in the memory. More specifically, the processor 102 of the apparatus 100 trains the image analysis system to analyse medical images using the set of training data stored in the memory. As mentioned earlier, the memory at which the set of training data is stored can be the memory 104 of the apparatus 100 or a memory external to the apparatus 100. It will thus be understood that any reference herein to the memory 104 can refer to the memory of the apparatus 100 or a memory external to the apparatus 100.

In some embodiments, the set of training data stored in the memory may comprise at least one medical image and one or more dependent variables (or target or output variables) associated with at least one medical image. The one or more dependent variables can, for example, be indicative of a property associated with the at least one medical image. According to some embodiments, the one or more dependent variables may be expressed as a value (for example, a positive or negative integer value or a yes/no value) indicative of the property associated with the at least one medical image. Thus, herein, the references to one or more dependent variables can be one or more dependent variable values according to some embodiments. Examples for a type of dependent variable include, but are not limited to, a continuous variable, a discrete variable, a categorical variable (for example, labels such as data labels), or any other dependent variable, or any combination of categorical variables associated with the at least one medical image. According to some embodiments, the dependent variables do not need to be independent of each other, nor do they need to be mutually exclusive or unambiguous. The one or more dependent variables are dependent in the sense that they can depend (i.e. vary depending) on the property associated with the at least one medical image.

For example, according to some embodiments, the one or more dependent variables can comprise any one or more of a dependent variable indicative of a disease (such as a pleural effusion, a cardiomegaly, an abnormal mediastinum, or any other disease of any other combination of diseases) identified in the at least one medical image, a dependent variable indicative of a defect identified in the at least one medical image, a dependent variable indicative of an object (such as an implant, one or more screws, a dental object, a pacemaker, a prostheses, or any other object, or any combination of objects) identified in the at least one medical image, a dependent variable indicative of an anatomical structure (such as a chest, a skull, one or more feet, one or more hands, one or more knees, an abdomen, a thorax, or any other anatomical, or any combination of anatomical structures) identified in the at least one medical image, a dependent variable indicative of an orientation of an anatomical structure identified in the at least one medical image, a dependent variable indicative of an orientation of the at least one medical image, or any other dependent variable, or any combination of dependent variables.

Returning back to Figure 2, at block 204, the user interface 106 is iteratively controlled to initiate the request for user feedback on the set of training data stored in the memory. More specifically, the processor 102 of the apparatus 100 iteratively controls the user interface 106 to initiate the request. As mentioned earlier, the user interface 106 that is controlled to initiate the request can be the user interface 106 of the apparatus 100 or a user interface external to the apparatus 100. It will thus be understood that any reference herein to the user interface 106 can refer to the user interface of the apparatus 100 or a user interface external to the apparatus 100. By iteratively controlling the user interface 106 to initiate requests for user feedback, the processor 102 of the apparatus 100 can continually (for example, constantly) collect user feedback to keep itself updated.

In some embodiments, the initiated request for user feedback can comprise a request for one or more dependent variables to associate with at least one medical image of the set of training data stored in the memory. For example, the initiated request for user feedback can comprise a request for one or more dependent variables to associate with at least one medical image of the set of training data that does not currently have any dependent variables associated with it. In an embodiment where the one or more dependent variables are labels, for example, the initiated request for user feedback can comprise a request to the user to label data associated with the at least one medical image that is currently unlabelled.

Alternatively or in addition, the initiated request for user feedback can comprise a request to confirm one or more dependent variables associated with at least one medical image of the set of training data stored in the memory. For example, the initiated request for user feedback can comprise a request to check the correctness (or the validity) of one or more dependent variables. Thus, the processor 102 of the apparatus 100 can be configured to provide one or more uncertain or ambiguous dependent variables to a user and control the user interface 106 to initiate a request for validation of those dependent variables by the user. For example, this may occur where the processor 102 detects there is an error or discrepancy with one or more dependent variables. In an embodiment where the one or more dependent variables are labels, the initiated request for user feedback can comprise a request to check the correctness (or validity) of one or more data labels associated with at least one medical image. In this way, by checking one or more dependent variables that are already associated with at least one image, the processor 102 of the apparatus 100 can reach a certain degree of redundancy and the reliability of the dependent variables associated with images can be improved.

Alternatively or in addition, the initiated request for user feedback can comprise a request to correct one or more dependent variables associated with at least one medical image of the set of training data stored in the memory. Thus, for example, user feedback can be collected for at least one medical image that already has one or more dependent variables associated it. More specifically, the initiated request for user feedback can comprise a request for a user provide feedback on one or more erroneous dependent variables associated with at least one medical image of the set of training data stored in the memory.

Alternatively or in addition, in some embodiments, the processor 102 of the apparatus 100 may generate synthetic cases (for example, internally). In these embodiments, a request may be initiated for user feedback on one or more dependent variables that are synthetically associated with at least one medical image of the set of training data stored in the memory. In this way, the processor 102 of the apparatus 100 has the ability to compensate for user-specific biases.

In some embodiments, the processor 102 of the apparatus 100 may be more likely to control the user interface 106 to initiate a request for user feedback on medical images of the set of training data stored in the memory 104 that do not have dependent variables associated with them or that have conflicting dependent variables associated with them. For example, the processor 102 of the apparatus 100 may be set to more often control the user interface 106 to initiate a request for user feedback on these medical images than others (where the others may be those that have non-conflicting or consistent dependent variables associated with them). In this way, the confidence level (i.e. the degree of certainty) for the retrained image analysis system can be increased, thereby increasing the accuracy of any image analysis that is subsequently performed using the retrained image analysis system. As such, the results (such as predictions, classifications, regression results, or any other type of results) that may be provided by the retrained image analysis system are more reliable.

Therefore, the processor 102 of the apparatus 100 may initiate a request for user feedback in a manner of ways. Although some examples have been provided for the type of request that is initiated for user feedback, it will be understood that any other request for user feedback on the set of training data, or any combination of requests for user feedback on the set of training data, are equally possible.

Returning back to Figure 2, at block 206, the set of training data stored in the memory 104 is updated based on user feedback received from the user interface 106 in response to the request. More specifically, the processor 102 of the apparatus 100 is configured to update the set of training data stored in the memory 104 based on user feedback received from the user interface 106 in response to the request.

Where the initiated request for user feedback comprises a request for one or more dependent variables to associate with at least one medical image of the set of training data stored in the memory 104, the user feedback received from the user interface in response to the request can comprise one or more dependent variables to associate with at least one medical image. Where the initiated request for user feedback comprises a request to confirm one or more dependent variables associated with at least one medical image of the set of training data stored in the memory 104, the user feedback received from the user interface in response to the request can comprise an indication of the correctness (or validity) of the one or more dependent variables. For example, the user feedback can comprise an indication that the one or more dependent variables are correct (or valid) or incorrect (or invalid). Where the initiated request for user feedback comprises a request to correct one or more dependent variables associated with at least one medical image of the set of training data stored in the memory 104, the user feedback received from the user interface in response to the request can comprise an indication of the correct one or more dependent variables associated with the at least one medical image.

Therefore, the user may interact with the apparatus 100 to provide feedback in a manner of ways. Although some examples have been provided for the type of user feedback, it will be understood that any other user feedback on the set of training data, or any combinations of user feedback on the set of training data, is equally possible.

In some embodiments, in addition to the user feedback that is received from the user interface in response to the request for user feedback, other user input may also be received by the processor 102 of the apparatus 100 from the user interface 106. For example, the user may request that the processor 102 of the apparatus 100 predicts one or more dependent variables of at least one medical image of the set of training data that does not currently have any dependent variables associated with it. In an embodiment where the one or more dependent variables are labels, for example, the user may request the processor 102 of the apparatus 100 to predict the label of a currently unlabelled medical image. Alternatively or in addition, the user may request that the processor 102 of the apparatus provide one or more (or all) medical images stored in the memory 104 that match one or more dependent variables. In an embodiment where the one or more dependent variables are labels, for example, the user may request the processor 102 of the apparatus 100 to provide one or more (or all) medical images stored in the memory 104 that match one or more given labels.

Although not illustrated in Figure 2, in some embodiments, the processor 102 of the apparatus 100 can be configured to control the memory 104 of the apparatus 100 (for example, a local database) to store each user feedback that is received from the user interface 106. In this way, the processor 102 of the apparatus 100 has the ability to assess inter-operator and intra-operator differences and can adjust internal confidence measures accordingly.

At block 208 of Figure 2, the image analysis system is retrained based on the updated set of training data stored in the memory 104. More specifically, the processor 102 of the apparatus 100 is configured to retrain the image analysis system based on the updated set of training data stored in the memory 104.

In some embodiments, the processor 102 of the apparatus 100 may retrain the image analysis system by updating (or adapting) one or more parameters of the image analysis system over time. In this way, the one or more parameters of the image analysis system can be adapted to a growing set of training data, which may be collected previous to deployment of the image analysis system and/or during the lifecycle of the image analysis system. Examples of the one or more parameters of the image analysis system that can be adapted include, but are not limited to, a bias (or bias function) to the weight given to one or more dependent variables, a slope coefficient in respect of one or more layers of the image analysis system (such as one or more layers of a deep neural network of the image analysis system), or any other parameter of the image analysis system, or any combination of parameters of the image analysis system. As mentioned earlier, the one or more parameters of the image analysis system may also be referred to as one or more independent (or input or predictor) variables.

Alternatively or in addition, the processor 102 of the apparatus 100 may retrain the image analysis system by adding parts to or removing parts from the image analysis system, for example, during the lifecycle of the system. For example, a topology of the image analysis system can be extended or reduced based on the updated set of training data used to retrain the image analysis system. In some embodiments, the retraining of the image analysis system may be scheduled by a quantity (or extent) of the updates to the set of training data. For example, the retraining of the image analysis system may be scheduled by the quantity (or extent) of new associations between one or more medical images and one or more dependent variables, which may also be referred to as new medical image and dependant variable pairs.

In some embodiments, the image analysis system can be retrained (for example, adapted) in the manner described herein on-site to improve the overall performance of the image analysis system. In some embodiments, the processor 102 of the apparatus 100 may take into account local requirements and guidelines when retraining the image analysis system based on the updated set of training data stored in the memory 104.

Optionally in some embodiments, as illustrated at block 210 of Figure 2, the method can further comprise determining a confidence level for the retrained image analysis system. The confidence level may also be referred to as the degree of certainty. More specifically, the processor 102 of the apparatus 100 can be configured to determine the confidence level for the retrained image analysis system. In some embodiments, for example, the processor 102 of the apparatus 100 can be configured to analyse one or more initial (or test) medical images using the retrained image analysis system to assess the current performance of the retrained image analysis system. For example, the one or more initial (or test) medical images that are analysed using the retrained image analysis system may be provided with and without dependent variables associated with them in order to assess the current performance of the retrained image analysis system. In this way, the processor 102 of the apparatus 100 can avoid over-fitting by the retrained image analysis system.

According to some embodiments, the processor 102 of the apparatus 100 can be configured to determine the confidence level for the retrained image analysis system by determining a probabilistic estimate of a degree of confidence in the retrained image analysis system. In some embodiments, the degree of confidence in the retrained image analysis system may be a degree of confidence in a predictive performance of the retrained image analysis system. Thus, the processor 102 of the apparatus 100 may maintain a probabilistic estimate of its own degree of confidence in the prediction. The predictive performance of the retrained image analysis system can be a measure on a set of validation cases that form an initial configuration of the retrained image analysis system, as described earlier.

In some embodiments where a confidence level is determined for the retrained image analysis system, if the confidence level is determined to be below a minimum threshold, the user interface 106 may be controlled to output a further request for user feedback associated with the set of training data stored in the memory 104 (as illustrated at block 212 of Figure 2). More specifically, the processor 102 of the apparatus 100 can be configured to control the user interface 106 to output the further request for user feedback. The confidence level may be based on an uncertainty in the image analysis (for example, the predictions) provided by the image analysis system. For example, the greater the uncertainty, the lower the confidence level (or, similarly, the lower the uncertainty, the greater the confidence level). Alternatively or in addition, the confidence level may be based on a training (or testing) error of the image analysis system. For example, the higher the error, the lower the confidence level (or, similarly, the lower the error, the higher the confidence level).

The further request for user feedback may be any one of or any combination of the requests for user feedback mentioned earlier, any other request for user feedback, or any other combination of requests for user feedback. In an embodiment where the one or more dependent variables comprise a label, for example, the processor 102 of the apparatus 100 may decide to query the label such as by initiating a request for the advice of a local user. By initiating the further request for user feedback where the confidence level drops below the minimum acceptable threshold, the confidence level achieved for the method and apparatus described herein can be improved.

The image analysis system retrained using the method described herein can operate automatically (for example, without human interaction) since it is continually ensured that the retrained image analysis system is optimised in the manner described herein.

In any of the embodiments described herein, as illustrated at block 214 of Figure 2, the method may optionally also comprise analysing one or more medical images using the retrained image analysis system. More specifically, the processor 102 of the apparatus 100 can be configured to analyse one or more medical images using the retrained image analysis system. The processor 102 of the apparatus 100 may be configured to analyse one or more medical images using the retrained image analysis system by way of any suitable image analysis technique. For example, in some embodiments, the processor 102 of the apparatus 100 can be configured to analyse the one or more medical images by assigning a classification to one or more medical images using the retrained image analysis system. Alternatively or in addition, in some embodiments, the processor 102 of the apparatus 100 can be configured to analyse the one or more medical images by performing a regression analysis on one or more medical images using the retrained image analysis system. However, although examples have been provided for the type of image analysis that may be performed, it will be understood that any form of image analysis may be performed on the one or more medical images using the retrained image analysis system.

Although not illustrated in Figure 2, in some embodiments, the processor 102 of the apparatus 100 may (for example, iteratively) control the user interface 106 to initiate a request for user feedback on one or more results, where the results are those provided by analysing one or more medical images using the retrained image analysis system. As mentioned earlier, the results may, for example, comprise one or more predictions, one or more classifications, one or more regression results, one or more machine learning results, or any other results, or any combination of results, that the image analysis system is suitable of providing. In some embodiments, the results for which a request for user feedback is initiated may be one or more uncertain or unambiguous results. For example, this may occur where the processor 102 detects there is an error or discrepancy with one or more results. In some embodiments, the results for which a request for user feedback is initiated may be a sample of results.

In embodiments where one or more medical images are analysed, the processor 102 of the apparatus 100 may also be configured to select (or predict) one or more parameters for image processing to apply to at least one medical image based on the analysis of the at least one medical image using the retrained image analysis system. For example, the retrained image analysis system can be used to identify one or more dependent variables associated with the at least one medical image (for example, an anatomical structure in the at least one medical image and/or its orientation in the at least one medical image) and this information can be used to select (or predict) the one or more image processing parameters to apply to at least one medical image, such as the one or more image processing parameters that are optimum for a given clinical task. Alternatively or in addition, in embodiments where one or more medical images are analysed, the processor 102 of the apparatus 100 may be configured to detect and name one or more anatomical structures or other objects in at least one medical image based on the analysis of the at least one medical image using the retrained image analysis system.

Alternatively or in addition, in embodiments where one or more medical images are analysed, the processor 102 of the apparatus 100 may be configured to adjust at least one medical image based on the analysis of the at least one medical image using the retrained image analysis system. For example, the retrained image analysis system can be used to identify one or more dependent variables associated with the at least one medical image (for example, an anatomical structure in the at least one medical image and/or its orientation in the at least one medical image) and this information can be used to select one or more adjustments to apply to at least one medical image, such as one or more adjustments that are optimum for a given clinical task. In some embodiments, the processor 102 of the apparatus 100 may be configured to predict one or more adjustments to apply to the at least one medical image based on the analysis of the at least one medical image using the retrained image analysis system and then subsequently apply the predicted adjustment to the at least one image.

In some embodiments, the processor 102 can be configured to adjust the at least one of the medical image by rotating the at least one medical image to alter an orientation of the at least one medical image based on the analysis of the at least one medical image using the retrained image analysis system. In these embodiments, for example, the dependent variable may be a continuous variable indicative of the orientation of the at least one image (for example, 39.976 degrees). In some embodiments, the orientation of the at least one medical image may be altered irrespective of the orientation of the at least one medical image during acquisition. For example, the orientation of the at least one medical image may be altered (for example, automatically) by rotating the at least one medical image to an "upright" orientation, regardless of its orientation during acquisition. As mentioned earlier, in some embodiments, the processor 102 of the apparatus 100 may be configured to predict one or more adjustments to apply to the at least one medical image based on the analysis of the at least one medical image. Thus, in these embodiments, the processor 102 of the apparatus 100 may be configured to predict a rotation angle adjustment to apply to the at least one medical image based on the analysis of the at least one medical image and then subsequently apply the predicted rotation angle adjustment to the at least one image.

Alternatively or in addition, in some embodiments, the processor 102 can be configured to adjust the at least one of the medical image by translating (or shifting) the least one medical image to alter a position of the at least one medical image in space based on the analysis of the at least one medical image using the retrained image analysis system. In these embodiments, for example, the dependent variable may be a discrete variable indicative of the translation (or shift) of the at least one image (for example, up, down, left, right). As mentioned earlier, in some embodiments, the processor 102 of the apparatus 100 may be configured to predict one or more adjustments to apply to the at least one medical image based on the analysis of the at least one medical image. Thus, in these embodiments, the processor 102 of the apparatus 100 may be configured to predict a translation adjustment to apply to the at least one medical image based on the analysis of the at least one medical image and then subsequently apply the predicted translation adjustment to the at least one image.

Although some examples have been provided for the type of adjustment to at least one medical image, it will be understood that any other adjustment and any combination of adjustments to at least one medical image equally possible.

In some embodiments, the processor 102 of the apparatus 100 may be configured to autonomously adjust at least one medical image based on the analysis of the at least one medical image using the retrained image analysis system. Thus, workflow components can be automated according to some embodiments. For example, in some embodiments, the processor 102 of the apparatus 100 may be configured to autonomously change an orientation (i.e. re-orientate) and/or change a processing of a displayed medical image depending on the contents (for example, an anatomy, or anatomical structure) of the medical image. In this way, the method described herein can allow for workflow automation.

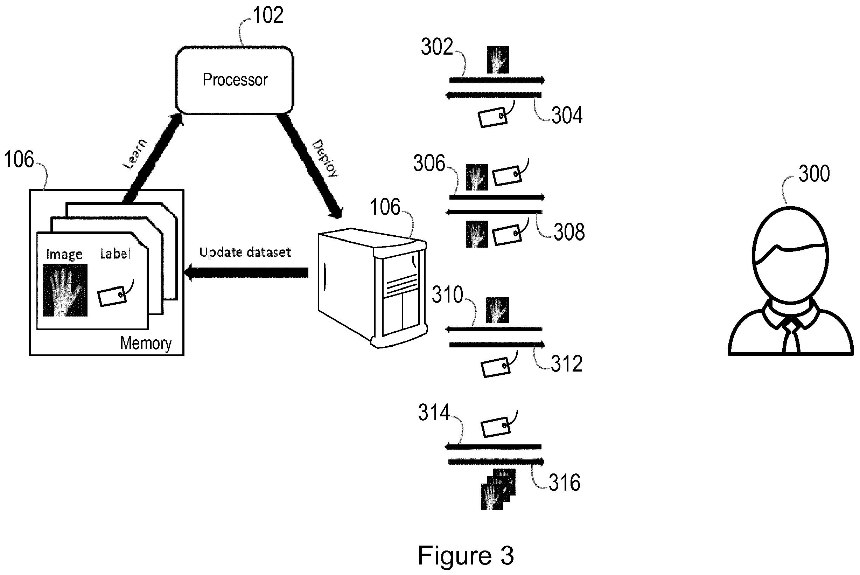

**Figure 3** illustrates the apparatus for training an image analysis system in use according to an embodiment. The processor 102 of the apparatus 100 shown in use in Figure 3 is configured to perform the method described above with reference to blocks 202, 204, 206 and 208 of Figure 3.

In particular, as illustrated in Figure 3, the processor 102 of the apparats 100 trains an image analysis system to analyse medical images using a set of training data stored in a memory 104 (which is indicated by the "Learn" arrow in Figure 3), iteratively controls a user interface 106 to initiate a request 302, 306 for user feedback on the set of training data stored in the memory (which is indicated by the "Deploy" arrow in Figure 3), updates the set of training data stored in the memory 104 based on user feedback 304, 308 received from the user interface in response to the request (which is indicated by the "Update dataset" arrow in Figure 3), and retrains the image analysis system based on the updated set of training data stored in the memory (which is again indicated by the "Learn" arrow in Figure 3). In other words, the method described above with reference to blocks 202, 204, 206 and 208 of Figure 2 are performed, which will not be repeated here but will be understood to reply.

As mentioned earlier with reference to Figure 2, a user 300 may interact with the apparatus 100 to provide user feedback in a manner of ways. Examples of the ways in which the user 300 may interact with the apparatus 100 are illustrated in Figure 3.

For example, as illustrated in Figure 3, the processor 102 of the apparatus 100 may initiate a request for user feedback on the set of training data stored in the memory 104, where the initiated request 302 comprises a request for one or more dependent variables to associate with at least one medical image of the set of training data stored in the memory 104. In this case, the user feedback 304 that is received by the processor 102 of the apparatus 100 from the user interface 106 in response to the request may be one or more dependent variables to associate with at least one medical image of the set of training data stored in the memory 104.

Alternatively or in addition, as illustrated in Figure 3, the processor 102 of the apparatus 100 may initiate a request for user feedback on the set of training data stored in the memory, where the initiated request 306 comprises a request to confirm one or more dependent variables associated with at least one medical image of the set of training data stored in the memory. In this case, the user feedback 308 that is received by the processor 102 of the apparatus 100 from the user interface 106 in response to the request may be an indication of the correctness of the one or more dependent variables (for example, an indication that the one or more dependent variables are correct or incorrect).

In some embodiments, in addition to the user feedback that is received from the user interface 106 in response to the request, other user input 310, 314 may be received by the processor 102 of the apparatus 100 from the user interface 106. For example, the other user input may be a user input 310 of one or more dependent variables that match at least one image. In this case, the processor 102 of the apparatus 100 may be configured to control the user interface 106 to render (or output or provide) one or more dependent variables 312 that match the at least one image. In another example, the other user input may comprise a user initiated request 314 that queries the processor 102 of the apparatus 100 for the same or a similar image to at least one image. In this case, the processor 102 of the apparatus 100 may be configured to control the user interface 106 to render (or output or provide) images 316 that are the same as or similar to the at least one image.

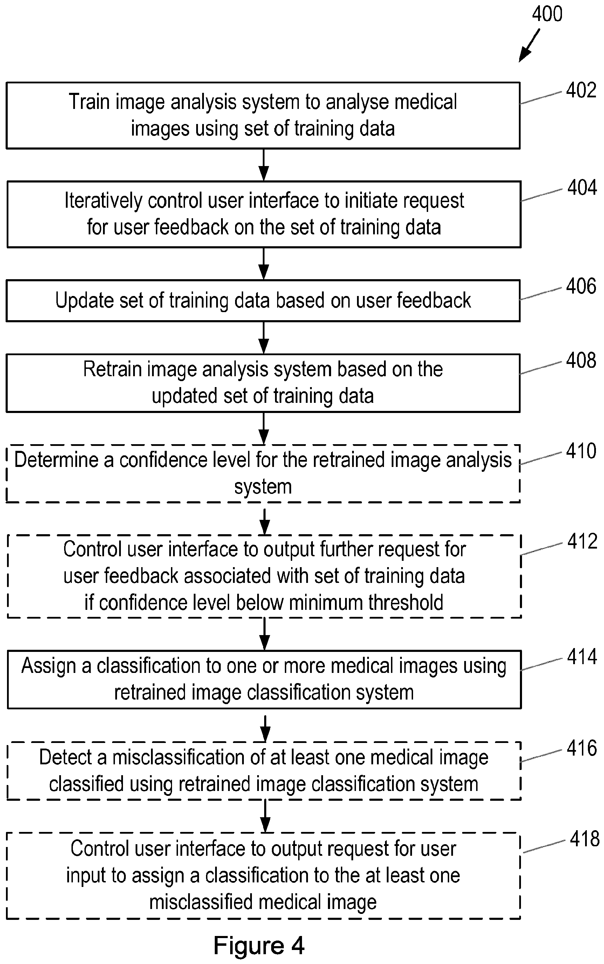

**Figure 4** illustrates a method 400 of operating an apparatus comprising a processor to train an image analysis system according to an example embodiment. In this example embodiment, the image analysis system is an image classification system. The illustrated method 400 of Figure 4 can generally be performed by or under the control of the processor 102 of the apparatus 100.

With reference to Figure 4, at blocks 402, 404, 406 and 408, the method described above with reference to blocks 202, 204, 206 and 208 of Figure 2 respectively is performed, which will not be repeated here but will be understood to apply. Optionally, at blocks 410 and 412 of Figure 4, the method described above with reference to blocks 210 and 212 of Figure 2 respectively is performed, which again will not be repeated here but will be understood to apply.

At block 414 of Figure 4, the one or more medical images are analysed by assigning a classification to one or more medical images using the retrained image classification system. More specifically, the processor 102 of the apparatus 100 is configured to analyse the one or more medical images by assigning a classification to one or more medical images using the retrained image classification system.

Optionally, at block 416 of Figure 4, a misclassification of at least one medical image classified using the retrained image classification system may be detected. More specifically, the processor 102 of the apparatus 100 can be configured to detect the misclassification of at least one medical image classified using the retrained image classification system. Optionally, at block 418 of Figure 4, the user interface 106 is controlled to output a request for a user input to assign a classification to the at least one misclassified medical image if a misclassification of at least one medical image classified using the retrained image classification system is detected. More specifically, the processor 102 of the apparatus 100 is configured to control the user interface 106 to output the request for the user input to assign a classification to the at least one misclassified medical image if a misclassification of at least one medical image classified using the retrained image classification system is detected. For example, the processor 102 of the apparatus 100 may initiate a request for a user input (e.g. a user opinion) on the particular class assigned to the at least one image.

Although not illustrated in Figures 2 or 4, in any of the embodiments described herein, the processor 102 of the apparatus 100 may be further configured to receive at least one (new) medical image, one or more dependent variables associated with at least one medical image, or both at least one (new) medical image and one or more dependent variables associated with the at least one medical image. In these embodiments, the processor 102 of the apparatus 100 can be configured to control the memory 104 to add the at least one received medical image and/or the one or more received dependent variables associated with the at least one medical image to the set of training data stored in the memory. In this way, new data or information can be actively collected and dependent variables can be added to the set of training data, for example, during the lifetime of the image analysis system. The new dependent variables that can be added to the set of training data can, for example, include new diseases, new objects, new categories of anatomical structure, new orientations of anatomical structures (such as any of those mentioned earlier), or any other new dependent variables.

In some embodiments, at least one (new) medical image may be received without dependent variables associated with the at least one medical image. In these embodiments, the processor 102 of the apparatus 100 can be configured to predict one or more dependent variables to be associated with the at least one medical image using the trained (or retrained) image analysis system. In some embodiments, the one or more predicted dependent variables may be corrected by a user via the user interface 106. Alternatively or in addition to predicting one or more variables, the processor 102 of the apparatus 100 can be configured to control the user interface 106 to request one or more dependent variables to associate with the at least one medical image. In other embodiments, the at least one medical image may be received with one or more dependent variables already associated with the at least one medical image. In any of these embodiments, the processor 102 of the apparatus 100 can be configured to control the memory 104 to add the at least one received medical image and the one or more dependent variables associated with the at least one medical image to the set of training data stored in the memory. In this way, the set of training data can be enlarged.

Thus, it is possible to add medical images and/or associated dependent variables to the set of training data stored in the memory 104. In other words, the medical images and/or associated dependent variables become part of the updated set of training data. The processor 102 of the apparatus 100 may be configured to retrain the image analysis system based on the added medical images and/or associated dependent variables. In this way, these medical images can be analysed by the retrained image analysis system in the correct manner. For example, where the retrained image analysis system is a retrained image classification system, the user can add categories and these categories are correctly classified by the retrained image classification system.

In some embodiments, the processor 102 of the apparatus 100 may acquire (or pool) information from one or more other image analysis systems (such as image analysis systems at different locations, which may have a different base of users). For example, the information can be at least one medical image with one or more associated dependent variables (such as at least one labelled medical image) that have been trained using the one or more other image analysis systems. The one or more other image analysis systems may, for example, train the one or more dependent variables associated with the at least one medical image until a certain level of confidence is achieved before the information is acquired by the processor 102 of the apparatus 100. Once acquired by the processor 102, the acquired information can then be propagated to the retrained image analysis system. Thus, in some embodiments, the information acquired from one or more other image analysis systems can also be used to retrain the image analysis system. In some embodiments, the processor 102 of the apparatus 100 may only propagate the acquired information to the retrained image analysis system after validating and/or consolidating (for example, aggregating and averaging) the information.

There is therefore provided an improved method and apparatus for training an image analysis system. For example, the method and apparatus described herein can provide an active (machine) learning system having a constantly improving accuracy. More specifically, according to the method and apparatus described herein, an image analysis system can be refined or fine-tuned to one or more dependent variables. In embodiments where the image analysis system comprises at least one network (such as at least one deep neural network), the at least one network can be refined or fine-tuned in this way according to the method described and apparatus herein. In this way, it is possible to achieve a higher confidence level in the image analysis system that is retrained in the manner described herein. Also, according to some embodiments, the method and apparatus described herein not only allows for an iterative adaption of the image analysis system over time (for example, in order to improve the performance of the image analysis system) but can also address user-specific analysis tasks, such as user-specific classification and regression tasks. The one or more dependent variables can, for example, be relevant for medical tasks and thus the refinement provided can facilitate workflow and reduce user interactions.

In an example, medical image processing and structure enhancement parametrisations may vary depending on the clinical task that causes the medical investigation. The processing of medical images may require manual user interactions to select the proper processing parametrisations for the given clinical task. An image classification and regression method based on the image analysis system retrained in the manner described herein can identify the imaged anatomy (for example, autonomously) and can help the user to pre-select the appropriate image processing and structure enhancement parametrisations.

In another example, the orientation of digital, portable detectors during image acquisition may lead to unintended upside down presentations of the examined anatomy. This may require manual user interactions to rotate the anatomy in an orientation preferred by the radiologists (for example, a perpendicular orientation). An image classification and regression method based on the image analysis system retrained in the manner described herein can be used to determine the rotation required for (for example, automatic) alignment of the given image to facilitate the workflow of the radiographer.

In another example, medical image processing and structure enhancement parametrisations may require adjustments in case of large high-dose and low-dose regions in a medical images (for example, caused by implants or direct radiation). The image analysis system retrained in the manner described herein can be used to determine the occurrence and/or location of high-dose and low-dose regions to select the appropriate image processing parametrisation.

The method and apparatus described herein can be used in any medical imaging device, apparatus or system, such as in a picture archiving and communication system (PACS), a vendor Neutral Archive (VNA), a radiological information system (RIS), radiology workstations, machine operator panels, or any other type of medical imaging device, apparatus or system.

There is also provided a computer program product comprising a computer readable medium, the computer readable medium having computer readable code embodied therein, the computer readable code being configured such that, on execution by a suitable computer or processor, the computer or processor is caused to perform the method or methods described herein.

Variations to the disclosed embodiments can be understood and effected by those skilled in the art, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. An apparatus (100) for training an image analysis system, the apparatus (100) comprising a processor (102) configured to:
train an image analysis system to analyse medical images using a set of training data stored in a memory (104);
iteratively control a user interface (106) to output a request for user feedback associated with the set of training data stored in the memory (104);
update the set of training data stored in the memory (104) based on user feedback received from the user interface (106) in response to the request; and
retrain the image analysis system based on the updated set of training data stored in the memory (104).

2. An apparatus (100) as claimed in claim 1, wherein the set of training data comprises:
at least one medical image; and
one or more dependent variables associated with at least one medical image, wherein the one or more dependent variables are indicative of a property associated with the at least one medical image.

3. An apparatus (100) as claimed in any one of claims 1 or 2, wherein the initiated request for user feedback comprises any one or more of:
a request for one or more dependent variables to associate with at least one medical image of the set of training data stored in the memory (104);
a request to confirm one or more dependent variables associated with at least one medical image of the set of training data stored in the memory (104); and
a request to correct one or more dependent variables associated with at least one medical image of the set of training data stored in the memory (104),
wherein the one or more dependent variables are indicative of a property associated with the at least one medical image.

4. An apparatus (100) as claimed in any one of the preceding claims, wherein the processor (102) is configured to:
determine a confidence level for the retrained image analysis system.

5. An apparatus (100) as claimed in claim 4, wherein the processor (102) is configured to:
control the user interface (106) to output a further request for user feedback associated with the set of training data stored in the memory (104) if the determined confidence level for the retrained image analysis system is determined to be below a minimum threshold.

6. An apparatus (100) as claimed in any one of the preceding claims, wherein the processor (102) is configured to:
analyse one or more medical images using the retrained image analysis system.

7. An apparatus (100) as claimed in claim 6, wherein the processor (102) is configured to:
select one or more parameters for image processing to apply to at least one medical image based on the analysis of the at least one medical image using the retrained image analysis system.

8. An apparatus (100) as claimed in any one of claims 6 or 7, wherein the processor (102) is configured to:
adjust at least one medical image based on the analysis of the at least one medical image using the retrained image analysis system.

9. An apparatus (100) as claimed in claim 8, wherein the processor (102) is configured to adjust the at least one of the medical image by:
rotating the least one medical image to alter an orientation of the at least one medical image based on the analysis of the at least one medical image using the retrained image analysis system.

10. An apparatus (100) as claimed in any one of claims 6 to 9, wherein the processor (102) is configured to analyse the one or more medical images by:
assigning a classification to one or more medical images using the retrained image classification system; and/or
performing a regression analysis on one or more medical images using the retrained image classification system.

11. An apparatus (100) as claimed in claim 10, wherein the processor (102) is configured to:
analyse the one or more medical images by assigning a classification to one or more medical images using the retrained image classification system;
detect a misclassification of at least one medical image classified using the retrained image classification system; and
control the user interface to output a request for a user input to assign a classification to the at least one misclassified medical image.

12. An apparatus (100) as claimed in any one of the preceding claims, wherein the processor (102) is further configured to:
receive at least one medical image and one or more dependent variables associated with the at least one medical image; and
control the memory (104) to add the at least one received medical image and the one or more received dependent variables associated with the at least one medical image to the set of training data stored in the memory (102),
wherein the dependent variables are indicative of a property associated with the at least one medical image.

13. An apparatus (100) as claimed in any one of the preceding claims, wherein the image analysis system comprises at least one deep neural network.

14. A method (200, 400) of operating an apparatus (100) comprising a processor (102) to train an image analysis system, the method (200, 400) comprising:
training (202, 402) an image analysis system to analyse medical images using a set of training data stored in a memory (104);
iteratively controlling (204, 404) a user interface to initiate a request for user feedback on the set of training data stored in the memory;
updating (206, 406) the set of training data stored in the memory (104) based on user feedback from the user interface (106) received in response to the request; and
retraining (208, 408) the image analysis system based on the updated set of training data stored in the memory (104).

15. A computer program product comprising a computer readable medium, the computer readable medium having computer readable code embodied therein, the computer readable code being configured such that, on execution by a suitable computer or processor, the computer or processor is caused to perform the method as claimed in claim 14.
